# EUROPEAN PATENT APPLICATION

(11) **EP 3 154 018 A1**
(43) Date of publication of application: **12.04.2017**
(21) Application number: 15803767.1
(22) Date of filing: 13.01.2015
(51) Int. Cl.: G06Q 50/22

(54) **METHOD, DEVICE AND SYSTEM FOR IMPROVING HEALTH CONDITION**

(30) Priority: 05.06.2014 CN 201410246960
(71) Applicant: Launch Tech Company Limited, Shenzhen, Guangdong 518129 (CN)
(72) Inventor: LIU, Jun, Shenzhen Guangdong 518129 (CN); CHEN, Ming, Shenzhen Guangdong 518129 (CN)
(74) Representative: MERH-IP Matias Erny Reichl Hoffmann Patentanwälte PartG mbB
(86) International application number: PCT/CN2015/070640
(87) International publication number: WO 2015/184806

(57) **Abstract**

The present invention is applied to the technical field of medical information, and provides a method, device, and system for improving health condition. The method comprises obtaining health data of a user and creating a health improvement plan according to the health data. In the present invention, when the user terminal obtains the health data of the user, it creates a health improvement plan that can direct the user to improve his/her health according to the health data, so that the user can manage his/her own health systematically and comprehensively, and health improvement plans such as scientific prescriptions, reasonable diet, correct exercise, good attitudes, and beautiful environments are brought into the life style of every person.

## Description

### FIELD OF THE INVENTION

The present invention relates to the technical field of medical information, and more particularly to a method, device, and system for improving health condition.

### BACKGROUND OF THE INVENTION

With the improvement of the living standard and the acceleration of the pace of life, the people are getting higher and higher awareness of their own health, many persons desire to track and manage their own health in a simple, quick, and efficient way, thereby preventing the occurrence of diseases and protecting themselves better.

With the growing popularity of mobile devices such as smart phones, iPads, and notebook computers, the people can use the mobile devices to manage their own health comprehensively, consult health knowledge at any time, understand their physical conditions by physical examinations at any time, and record their own health experience at any time. In this way, the requirements for preventing diseases and always paying attention to own health of the people are met well, and much time and medical cost of the people are saved.

At present, there are many kinds of applicant software relating to health on the application market, and these kinds of software generally emphasize one aspect or several aspects relating to health to manage individual health. Some of the software emphasizes the function of searching and reading health knowledge such as health knowledge relating to health preservation, diet, weight loss, health care, folk prescriptions, secret prescriptions, and commonly used prescriptions; some of the software emphasizes some health problems of certain people, for example, weight loss, allows users to customize weight loss programs individually, and provides recipe references; and some of the software emphasizes commercial marketing and provides less health functions and more commercial advertisements, so that users may be bothered.

It is the most important that most application software cannot obtain health data of a user at any time, and cannot provide a health improvement plan to the user according to the health data and enable the user to improve his/her own health according to the health improvement plan.

### SUMMARY OF THE INVENTION

Embodiments of the present invention provide a method, device, and system for improving health condition, which aim at solve the problems in the prior art that health data of a user cannot be obtained at any time, and it is unable to provide the user with a health improvement plan according to the health data so that the user can improve his/her own health according to the health improvement plan.

In one aspect, a method for improving health condition is provided, wherein the method comprises:
obtaining health data of a user; and
creating a health improvement plan according to the health data.

Preferably, after creating the health improvement plan according to the health data, the method further comprises:
generating a reminding message according to the health improvement plan, and reminding the user to perform the health improvement plan by the reminding message.

Preferably, the health data includes at least one of traditional Chinese medicine (TCM) physical examination data, mental status data, and health index data;
obtaining the health data of the user includes:
performing a TCM physical examination to obtain the TCM physical examination data; and/or
performing psychological tests in multiple versions to obtain the mental status data; and/or
communicating with a health index testing terminal to receive the health index data, wherein the health index data is obtained by a test performed by the health index testing terminal.

Preferably, the method further comprises:
sending a request for customizing health knowledge to a medical information server;
receiving health knowledge introduced by the medical information server; and/or
communicating with friends, doctors, or experts to share health knowledge via an instant messaging server.

In another aspect, a device for improving health condition is provided, wherein the device comprises:
a health data obtaining unit configured for obtaining health data of a user; and
an improvement plan creating unit configured for creating a health improvement plan according to the health data.

Preferably, the device further comprises:
a performing reminding unit configured for generating a reminding message according to the health improvement plan, and reminding the user to perform the health improvement plan by the reminding message.

Preferably, the health data includes at least one of TCM physical examination data, mental status data, and health index data;
the health data obtaining unit includes:
a TCM physical examination module configured for performing a TCM physical examination to obtain the TCM physical examination data; and/or
a psychological testing module configured for performing psychological tests in multiple versions to obtain the mental status data of the user; and/or
a health index obtaining module configured for communicating with a health index testing terminal to receive the health index data, wherein the health index data is obtained by a test performed by the health index testing terminal.

Preferably, the device further comprises:
a customizing request sending unit configured for sending a request for customizing health knowledge to a medical information server;
a health knowledge receiving unit configured for receiving health knowledge introduced by the medical information server; and/or
a health knowledge sharing unit configured for communicating with friends, doctors, or experts to share health knowledge via an instant messaging server.

In a further aspect, a system for improving health condition is provided, wherein the system comprises a health index testing terminal and a user terminal, and the user terminal includes the above-described device for improving health condition.

Preferably, the system further comprises:
a medical information server;
a push server connected with the medical information server, wherein the push server is configured for retransmitting health knowledge introduced by the medical information server to the user terminal; and/or
an instant messaging server, wherein the instant messaging server is connected with the user terminal and configured for enabling a user of the user terminal to communicate with friends, doctors, or experts and share health knowledge.

In the embodiments of the present invention, when a user terminal obtains health data of a user, it creates a health improvement plan that can direct the user to improve his/her health according to the health data, so that the user can manage his/her own health systematically and comprehensively, and health improvement plans such as scientific prescriptions, reasonable diet, correct exercise, good attitudes, and beautiful environments are brought into the life style of every person.

### BRIEF DESCRIPTION OF THE DRAWINGS

Fig. 1 is a flow chart of a method for improving health condition provided by a first embodiment of the present invention.
Fig. 2 is a block diagram of a device for improving health condition provided by a second embodiment of the present invention.
Fig. 3 is a block diagram of a system for improving health condition provided by a third embodiment of the present invention.

### DETAILED DESCRIPTION OF THE PREFERRED EMBODIMENT

In order to make the purposes, technical solutions, and advantages of the present invention be clearer, the present invention will be further described in detail hereafter with reference to the accompanying drawings and embodiments. It should be understood that the embodiments described herein are only intended to illustrate but not to limit the present invention.

In the embodiments of the present invention, when a user terminal obtains health data of a user, the user terminal creates a health improvement plan that can direct the user to improve his/her health according to the health data.

The realization of the present invention will be described in detail hereafter with reference to the embodiments.

### Embodiment I

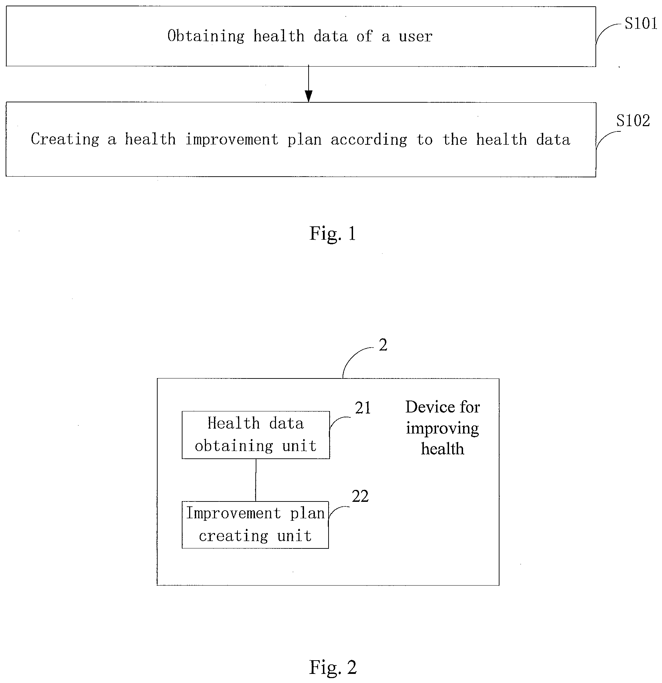

Fig. 1 shows a flow chart of a method for improving health condition provided by a first embodiment of the present invention, the method can be performed in a user terminal, and comprises the following steps.

Step S101: obtaining health data of a user.

In this embodiment, the health data of the user is physical examination data obtained after the user performs a physical examination, and includes at least one of traditional Chinese medicine (TCM) physical examination data obtained after the user performs a TCM physical examination, mental status data of the user, and health index data of the user.

In particular, the user can perform a TCM physical examination by the user terminal. When performing the TCM physical examination, aiming at every TCM physical examination question, the user selects a corresponding answer that is suitable for himself/herself, and the user terminal matches the answers of the user with a predetermined historical physical examination result database to obtain TCM physical examination data corresponding to the answers of the user.

In particular, the user can perform psychological tests in multiple versions by the user terminal, so that the mental status of the user can be understood comprehensively and the mental status data of the user can be obtained.

In particular, the user can obtain the health index data by a health index testing terminal, wherein the health index data can include the user's body weight data, blood pressure data, heart rate data, exercise history data, and so on.

Wherein, the health index testing terminal can include a body weight meter and a blood pressure meter, and can also include a smart wearable device such as a smart wristband; real-time data in daily life, such as exercise data, sleep data, diet data, and so on, can be recorded by the smart wearable device, and the smart wearable device can also synchronize the real-time data to the user terminal.

In particular, in this embodiment, the health index testing data establishes a communicating connection with the user terminal via a Bluetooth protocol.

Preferably, when the user terminal obtains the health data of the user, it can store the health data in a historical testing record database. The user terminal can read the historical testing record database, and the health data can be displayed to the user at the user terminal, such that the user can understand his/her own health.

Step S102: creating a health improvement plan according to the health data.

In this embodiment, when a medicine information server receives the health data sent from the user terminal, aiming at different physique and health, it creates a health improvement plan including drugs, food, exercise, mood, and environment.

In particular, the user terminal stores a comparison table configured for comparing the health data with the health improvement plan; when the user terminal receives the health data of the user, it matches the received health data with health data stored in the comparison table and thereby obtains a health improvement plan corresponding to the received health data. The user can be directed to live more healthily by the health improvement plan.

Preferably, the method further comprises the following steps.

Step S103: generating a reminding message according to the health improvement plan, and reminding the user to perform the health improvement plan by the reminding message.

In this embodiment, the health improvement plan include various time points, wherein these time points are prompt time points when the user should perform certain operations, for example, if the user should take medicine at one o'clock every day, when the user terminal detects that the time reaches one o'clock, it generates a reminding message to remind the user to take medicine.

Preferably, when the user terminal detects that the user has already performed the current health improvement plan completely, it reminds the user to perform a physical examination again, and creates a new health improvement plan according to health data obtained again. The user can continue to improve his/her own health according to the new health improvement health.

Preferably, the user terminal can send a request for customizing health knowledge to the medical information server, and receives health knowledge introduced by the medical information server.

Wherein, the user can read a large amount of professional health knowledge, which comprises various knowledge databases such as a medicine database, a food database, an exercise database, a mood database, an environment database, and so on, to increase his/her own health knowledge. In order to read the health knowledge, the user can customize some interesting health knowledge according to his/her interest.

In particular, the medicine information server can use a corresponding application programming interface (API) provided by a push server, and thereby push some customized health knowledge which is interesting to the user to the user terminal according to actual business requirements. All health knowledge introduced by the medical information server is sent to the user terminal by the push server.

In particular, the health knowledge includes a medicine database (e.g., comprising a drug database, a medicinal material database, a prescription database, a commonly used prescription database, a consilia database, and a symptom database), a food database (e.g., comprising an ingredient database and a recipe database), an exercise database, a mood database, an environment database, and so on.

Preferably, the user of the user terminal can communicate with friends, doctors, or experts and share health knowledge via an instant messaging (IM) server.

Wherein, the user terminal keeps a continuous socket connection state with the IM server. The IM server provides a corresponding service access interface used by the user terminal. By using an API provided by a third-party platform such as Wechat, Tencent Weibo, Sina Weibo, Renren network, and so on, the user sends real-time messages to friends, doctors, or experts via the IM server, and performs health communication with the friends, doctors, or experts. In particular, the user can communicate with the friends, doctors, or experts via the IM server to research his/her physical and mental diseases, discuss interactively, and ask for help; the user can also retransmit health experience to friends of his/her friend circle to share the health experience. Additionally, the user can also record health preservation experience and share the health preservation experience with his/her friends.

Preferably, the user can maintain personal data and historical testing records via the user terminal, and can further select to add some distinctive function modules.

Preferably, the user can perform other universal operations such as setting the display language, replacing the skin, updating the version, transferring his/her personal data and historical testing records to the cloud, downloading his/her historical testing records from the cloud to the local user terminal, and so on.

In this embodiment, when the user terminal obtains the health data of the user, it creates a health improvement plan that can direct the user to improve his/her health according to the health data, so that the user can manage his/her own health systematically and comprehensively, and health improvement plans such as scientific prescriptions, reasonable diet, correct exercise, good attitudes, and beautiful environments are brought into the life style of every person.

Furthermore, the user terminal can remind the user to perform the health improvement plan recommended to the user, and thus can prevent the user from forgetting to perform the health improvement plan. It is more humanized, and can further improve the use experience of the user.

Furthermore, when the user has already performed the current health improvement plan completely, the user terminal can remind the user to perform a physical examination again, and create a new health improvement plan according to the health data obtained by the new physical examination, thereby realizing continuous tracing for the health of the user.

Furthermore, the user terminal can further receive the health knowledge introduced by the medical information server, and can also communicate with friends, doctors, or experts to share health knowledge. The user terminal can realize various health management functions, can meet various health requirements of the user, and can save the network flow of the user and reduce the cost for access Internet.

One of ordinary skill in the art should understand that all or some of the steps of the method of the above-described embodiment can be accomplished by using programs to instruct relevant hardware, the relevant programs can be stored in a computer-readable storage medium, the storage medium can be ROM, RAM, a magnetic disk, a compact disc, and so on.

### Embodiment II

Fig. 2 shows a block diagram of a device for improving health condition provided by a second embodiment of the present invention. In order to facilitate the description, only the parts relating to this embodiment of the present invention are shown. The device for improving health condition can be a software unit, a hardware unit, or a unit combining software with hardware, and is built in the user terminal. Preferably, the user terminal can be a mobile device such as a smart phone, an iPad, a notebook computer, etc. The device 2 comprises a health data obtaining unit 21 and an improvement plan creating unit 22.

Wherein, the health data obtaining unit 21 is configured for obtaining health data of a user; and the improvement plan creating unit 22 is configured for creating a health improvement plan according to the health data.

Preferably, the device 2 further comprises:
a performing reminding unit configured for generating a reminding message according to the health improvement plan, and reminding the user to perform the health improvement plan by the reminding message.

In particular, the health data includes at least one of traditional Chinese medicine (TCM) physical examination data, mental status data, and health index data;
the health data obtaining unit includes:
a TCM physical examination module configured for performing a TCM physical examination to obtain the TCM physical examination data; and/or
a psychological testing module configured for performing psychological tests in multiple versions to obtain the mental status data of the user; and/or
a health index obtaining module configured for communicating with a health index testing terminal to receive the health index data, wherein the health index data is obtained by a test performed by the health index testing terminal.

Preferably, the device 2 further comprises:
a customizing request sending unit configured for sending a request for customizing health knowledge to a medical information server;
a health knowledge receiving unit configured receiving health knowledge introduced by the medical information server; and/or
a health knowledge sharing unit configured for communicating with friends, doctors, or experts to share health knowledge via an instant messaging (IM) server.

The device for improving health condition provided by this embodiment of the present invention can be used in the method of the aforementioned embodiment I correspondingly. The details of the use can refer to the description of the aforementioned embodiment I and are not repeated here.

### Embodiment III

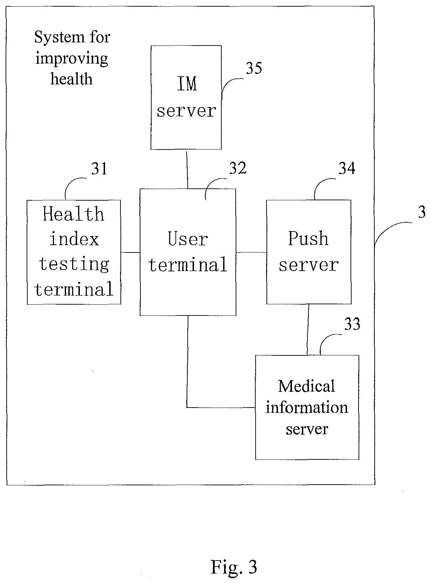

Fig. 3 shows a block diagram of a system for improving health condition provided by a third embodiment of the present invention. In order to facilitate the description, only the parts relating to this embodiment of the present invention are shown. The system 3 for improving health condition comprises a health index testing terminal 31 and a user terminal 32, and the user terminal 32 includes the device for improving health condition of the embodiment II.

The health index testing terminal 31 can collect health index data of a user and send the health index data to the user terminal 32, so that the user can read the health index data and adjust his/her physical and mental health according to the health index data in real-time.

According to at least one of the health index data, traditional Chinese medicine (TCM) physical examination data obtained by performing a TCM physical examination, and mental status data, the user terminal 32 can create a health improvement plan which can direct the user to improve his/her health.

Preferably, the user terminal 32 can generate a reminding message according to the health improvement plan and remind the user to perform the health improvement plan by the reminding message.

Preferably, the device 3 further comprises a medical information server 33 and a push server 34 connected with the medical information server 33, wherein the push server 34 is configured for retransmitting health knowledge introduced by the medical information server 33 to the user terminal 32.

Preferably, the device 3 further comprises an instant messaging (IM) server 35, wherein the IM server 35 is connected with the user terminal 32 and configured for enabling the user of the user terminal 32 to communicate with friends, doctors, or experts and share health knowledge via the IM server 35.

The system for improving health condition provided by this embodiment of the present invention can be used in the method of the aforementioned embodiment I correspondingly. The details of the use can refer to the description of the aforementioned embodiment I and are not repeated here.

It should be noted that the units included by the above-described system embodiment are divided only according to function logics, and the units are not limited to the above division, as long as they can realize corresponding functions; moreover, the specific names of the function units are only intended to be distinguished from each other easily but not to limit the protection scope of the present invention.

The above are only preferred embodiments of the present invention and are not intended to limit the present invention. Any modification, equivalent replacement, and improvement made within the spirit and principle of the present invention should be included in the protection scope of the present invention.

## Claims

1. A method for improving health condition, comprising:
obtaining health data of a user; and
creating a health improvement plan according to the health data.

2. The method according to claim 1, wherein after creating the health improvement plan according to the health data, the method further comprises:
generating a reminding message according to the health improvement plan, and reminding the user to perform the health improvement plan by the reminding message.

3. The method according to claim 1, wherein the health data includes at least one of traditional Chinese medicine (TCM) physical examination data, mental status data, and health index data;
obtaining the health data of the user includes:
performing a TCM physical examination to obtain the TCM physical examination data; and/or
performing psychological tests in multiple versions to obtain the mental status data; and/or
communicating with a health index testing terminal to receive the health index data, wherein the health index data is obtained by a test performed by the health index testing terminal.

4. The method according to claim 1, further comprising:
sending a request for customizing health knowledge to a medical information server;
receiving health knowledge introduced by the medical information server; and/or
communicating with friends, doctors, or experts to share health knowledge via an instant messaging server.

5. A device for improving health condition, comprising:
a health data obtaining unit configured for obtaining health data of a user; and
an improvement plan creating unit configured for creating a health improvement plan according to the health data.

6. The device according to claim 5, further comprising:
a performing reminding unit configured for generating a reminding message according to the health improvement plan, and reminding the user to perform the health improvement plan by the reminding message.

7. The device according to claim 5, wherein the health data includes at least one of traditional Chinese medicine (TCM) physical examination data, mental status data, and health index data;
the health data obtaining unit includes:
a TCM physical examination module configured for performing a TCM physical examination to obtain the TCM physical examination data; and/or
a psychological testing module configured for performing psychological tests in multiple versions to obtain the mental status data of the user; and/or
a health index obtaining module configured for communicating with a health index testing terminal to receive the health index data, wherein the health index data is obtained by a test performed by the health index testing terminal.

8. The device according to claim 5, further comprising:
a customizing request sending unit configured for sending a request for customizing health knowledge to a medical information server;
a health knowledge receiving unit configured for receiving health knowledge introduced by the medical information server; and/or
a health knowledge sharing unit configured for communicating with friends, doctors, or experts to share health knowledge via an instant messaging server.

9. A system for improving health condition comprising a health index testing terminal and a user terminal, wherein the user terminal includes the device for improving health condition of any one of claims 5-8.

10. The system according to claim 9, further comprising:
a medical information server;
a push server connected with the medical information server, wherein the push server is configured for retransmitting health knowledge introduced by the medical information server to the user terminal; and/or
an instant messaging server, wherein the instant messaging server is connected with the user terminal and configured for enabling a user of the user terminal to communicate with friends, doctors, or experts and share health knowledge.
